(19) Europäisches Patentamt
European Patent Office
Office européen des brevets

(11) Veröffentlichungsnummer : **0 057 809**
**B1**

(12) # EUROPÄISCHE PATENTSCHRIFT

(45) Veröffentlichungstag der Patentschrift : **18.06.86**

(51) Int. Cl.[4] : **G 01 N 33/84,** C 07 C121/75

(21) Anmeldenummer : **82100051.0**

(22) Anmeldetag : **07.01.82**

(54) **Verfahren zur Bestimmung des pH-Wertes im Innern einer Zelle.**

(30) Priorität : **06.02.81 DE 3104078**

(43) Veröffentlichungstag der Anmeldung : **18.08.82 Patentblatt 82/33**

(45) Bekanntmachung des Hinweises auf die Patenterteilung : **18.06.86 Patentblatt 86/25**

(84) Benannte Vertragsstaaten : **AT BE CH DE FR GB IT LI LU NL SE**

(56) Entgegenhaltungen :
**JOURNAL OF THE CHEMICAL SOCIETY, Faraday Trans. 1, Band 73, Nr. 9, 1977, Seiten 1281-1285 R.G. BROWN et al.: "Effect of pH on the emission and absorption characteristics of 2,3-dicyano-p-hydroquinone"**
**BIOCHEMISTRY, Band 18, Nr. 11, 29. Mai 1979, Seiten 2210-2218, USA J.A. THOMAS et al.: "Intracellular pH measurements in Ehrlich Ascites Tumor Cells utilizing spectroscopic probes generated in situ"**
**CHEMICAL ABSTRACTS, Band 90, Nr. 19, 7. Mai 1979, Seite 258, Nr. 148002f, Columbus, Ohio, USA, J.W.M. VISSER et al.: "Intracellular pH-determination by fluorescence measurements" (CHISSO CORP. OSAKA, JAPAN)**
**CHEMICAL ABSTRACTS, Band 93, Nr. 7, 18. August 1980, Seite 427, Nr. 65125j, Columbus, Ohio, USA, O.V. YARANTSEVA et al.: "Fluorescence method for the determination of cell pH using the phenomenon of "fluorochromasia"**
**Handbook of Chemistry and Physics, 54, Auflage (CRC-Press 1973-1974), S. D-117 und D-118**
**J. Chem. Soc. 1948, 303**

(73) Patentinhaber : **Max-Planck-Gesellschaft zur Förderung der Wissenschaften e.V.
Bunsenstrasse 10
D-3400 Göttingen (DE)**

(72) Erfinder : **Ruhenstroth-Bauer, Gerhard, Prof. Dr.
Spitzelbergerstrasse 11
D-8032 Gräfelfing (DE)**
Erfinder : **Wünsch, Erich, Prof. Dr.
Midgardstrasse 16
D-8132 Tutzing (DE)**
Erfinder : **Valet, Günter, Dr.
Gräfelfingerstrasse 79 a
D-8000 München (DE)**
Erfinder : **Moroder, Luis, Prof. Dr.
Lena-Christ-Strasse 12
D-8033 Martinsried (DE)**

(74) Vertreter : **Dreiss, Uwe, Dr. jur. Dipl.-Ing. M.Sc. et al
Patentanwälte Dreiss, Hosenthien & Fuhlendorf
Gerokstrasse 6
D-7000 Stuttgart 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 057 809 B1

**Beschreibung**

Die Erfindung betrifft ein Verfahren zur Bestimmung des pH-Wertes im Inneren einer Zelle durch Messung der Emission einer fluoreszierenden Substanz, die von der Zelle durch Inkubation in einer Lösung aufgenommen wird, die ein membrangängiges Derivat dieser Substanz enthält, und die in der Zelle durch enzymatische Spaltung aus diesem Derivat freigesetzt wird.

Ein derartiges Verfahren ist aus Chem. Abstr. 93 (1980), Referat 65125 j bekannt. Dabei wird die pH-abhängige Intensität der Fluoreszenz des Fluoresceins zur Messung des intrazellulären pH-Wertes ausgenützt (Fluorochromasie). Fluorescein ist Repräsentant der Mehrheit der bekannten Fluoreszenz-Indikatoren, bei denen sich pH-abhängig die Intensität des emmitierten Fluoreszenzlichtes ändert. Die Messung erfolgt entweder als Einzelzellmessung unter dem Mikroskop (Cytofluorometer) oder als Küvettenmessung (Fluorometer). Da die Intensität infolge unterschiedlicher intrazellulärer Anreicherung und unterschiedlicher intrazellulärer Mengen bei unterschiedlich großen Zellen kein Maß für das intrazelluläre pH ist, kann man den pH-Wert nur auf der Grundlage zweier aufeinanderfolgender Messungen der Intensität bei verschiedenen Anregungswellenlängen bestimmen. Das Verhältnis der beiden nacheinander gemessenen Intensitäten ist dann ein Maß für den pH-Wert im Inneren der Zelle.

Dabei ergeben sich jedoch eine Reihe von Schwierigkeiten. Im Falle der verhältnismäßig langsamen mikroskopischen Einzelfallmessung mit dem Cytofluorometer ergeben sich leicht Meßwertverfälschungen wegen des schnellen Ausbleichens der Fluoreszenz unter der intensiven Lichtbestrahlung. Dies gilt insbesondere für das Fluorescein. Aber auch bei der durch aufeinanderfolgende Messung mehrerer Zellen im Durchflußcytometer oder im Sellsorter vorgenommenen Bestimmung der Intensität der Fluoreszenz sind zwei nacheinanderfolgende Anregungen mit verschiedenen Laser-Lichtlinien erforderlich. Es besteht wiederum das Problem des Ausbleichens des Fluoresceins. Ferner ist in Form von elektronischen Koinzidenzschaltungen ein erheblicher apparativer Aufwand erforderlich, um sicherzustellen, daß die beiden Fluoreszenzsignale, die miteinander verglichen werden, auch tatsächlich von derselben Zelle stammen.

Es ist bekannt, den pH-Wert im Innern einer Zelle ($pH_i$) mit Hilfe von Elektroden, die man in die Zellen einsticht, elektrisch zu messen. Nachteilig ist dabei die teilweise Zerstörung der Zellwand, die Ungenauigkeit der Messung und die umständliche, zeitaufwendige Handhabung.

Es ist auch bekannt, Zellen, deren $pH_i$-Wert gemessen werden soll, in Fluoreszeinester-haltigen Lösungen zu inkubieren. Freies Fluoreszein durchdringt die Zellmembran nicht. Verestertes, nicht fluoreszentes Fluoreszein ist dagegen membrangängig. Der Ester wird nach dem Durchdringen der Zellwand von den in der Zelle vorhandenen Enzymen gespalten. Dadurch entsteht das ursprüngliche, fluoreszierende Fluoreszein, das die Zelle nicht mehr verlassen kann und sich deshalb im Zellinnern anreichert. Bei den im Zellinneren vorhandenen Enzymen, die diese Abspaltung (Hydrolyse) bewirken, handelt es sich um Esterasen. Der $pH_i$ kann nun dadurch bestimmt werden, daß man Zellen in einer photometrischen Küvette in einer Fluoreszeinester enthaltenden physiologischen Pufferlösung suspendiert, und diese Suspension alternierend mit zwei Lichtquellen unterschiedlicher Anregungswellenlänge beleuchtet, wobei man gleichzeitig die Intensität des Fluoreszenzlichtes bei der Frequenz des Emissions-Maximums misst (Thomas et al., Intracellular pH-Measurements in Ehrlich Ascites Tumor Cells Utilizing Spectroscopic Probes Generated in Situ, Biochemistry Vol. 18, No. 11, S. 2210-2218 (1979)). Dieses Verfahren hat den Nachteil, daß keine Einzelzellen gemessen werden können. Die Einzelzellmessung ist jedoch von großer Wichtigkeit, weil eine Zellsuspension mehrere Zellarten mit unterschiedlichem $pH_i$ enthalten kann, die mit der photometrischen Küvettenmethode nicht entdeckbar sind.

Es ist ferner bekannt, daß Zellsuspensionen, die nach vorstehender Weise mit Fluoreszeinestern behandelt wurden, in einem Durchflußcytometer gemessen werden können (Visser et al., Intracellular pH-Determination by Fluorescence Measurement, The Journal of Histochemistry and Cytochemistry, Vol. 27, No. 1, S. 32-35 (1979)), wo die Fluoreszenzemission von Einzelzellen bestimmt wird. Im Durchflußcytometer werden die Einzelzellen mit einer Lichtwellenlänge angeregt. Da eine $pH_i$ Bestimmung bei Verwendung von Fluoreszeinestern jedoch nur bei Messung der Fluoreszenz bei zwei Anregungswellenlängen möglich ist, sind zwei separate Messungen an derselben Zellsuspension notwendig (a.a.O. S. 34 li. Sp., Z. 4 und S. 35 li. Sp., Z 6). Das bedeutet, daß wie beim photometrischen Küvettenverfahren nur der Mittelwert des $pH_i$ aller gemessenen Zellen bestimmt werden kann, weil die Zuordnung der Meßwerte für die Einzelzelle bei zweimaligem Durchlauf der Zellsuspension durch das Durchflußcytometer nicht möglich ist.

Aufgabe der Erfindung ist es, sein Verfahren zu schaffen und die dazu erforderlichen Substanzen bereit zu stellen, durch die die Bestimmung des pH-Wertes im Innern von Einzelzellen möglich wird. Dieses Verfahren soll sich insbesondere auch für das Durchmessen ganzer Zellpopulationen in Durchflußcytometern eignen, so daß entsprechende Verteilungskurven in einfacher Weise erstellt werden können, die ihrerseits neue Möglichkeiten der Diagnostik eröffnen.

Diese Aufgabe wird dadurch gelöst, daß als fluoreszierende Substanz eine solche ausgewählt wird, bei der die Frequenz des Maximums der Emission pH-abhängig ist, und daß als Verbindung dieser fluoreszierenden Substanz eine solche eingesetzt wird, die membrangängig ist, und daß ferner bei der Messung der Fluoreszenz die pH-abhängige Frequenz bestimmt wird, bei der das Maximum der Fluoreszenzemission auftritt.

Das Wesentliche der Erfindung besteht also zunächst darin, als fluoreszierenden Indikator eine solche Substanz zu nehmen, bei der die Frequenz und nicht die Intensität der Fluoreszenz pH-abhängig ist. Damit ist gemeint, daß diejenige Frequenz pH-abhängig ist, bei der das Emissionsspektrum sein Maximum hat. Ferner ist wichtig, diese Substanz membrangängig zu machen, so daß sie aus der Lösung, in der die Zellen inkubiert werden, durch die Zellwand in das Zellinnere eintritt. Die Ausgangssubstanz ist dazu nicht geeignet. Nun ergibt sich allerdings, daß durch die zur Verbesserung des Durchtritts durch die Zellwand herbeigeführte Derivatisierung sich eine Verbindung ergibt, die ihrerseits nicht in der gewünschten Weise fluoresziert. Man muß also eine solche fluoreszierende Substanz als Ausgangsprodukt für die Derivatisierung nehmen, deren Verbindung membrangängig ist und die in der Zelle wieder durch ein dort vorhandenes Enzym so gespalten wird, daß im Zellinnern wieder die in der gewünschten Weise pH-abhängig fluoreszierende Substanz entsteht. Wegen der fehlenden Membrangängigkeit dieser fluoreszierenden Substanz kann sie die Zelle nicht mehr verlassen und reichert sich im Zellinnern an, was Grundlage für die Messung ist.

Gemäß der Erfindung vermeidet man den Nachteil der sequentiellen Intensitätsmessung der Fluoreszenz bei zwei verschiedenen Anregungswellenlängen, wie dies bei Fluoreszeinestern zur $pH_i$ Bestimmung erforderlich ist und wodurch die $pH_i$ Bestimmung nur an Zellsuspensionen und nicht für einzelne Zellen durchgeführt werden kann, dadurch, daß Substanzen mit pH-abhängigem Emissionsspektrum verwendet werden. Die Vorteile sind, daß die $pH_i$ Bestimmung in Einzelzellen möglich wird, daß nur eine Anregungswellenlänge erforderlich ist und daß die Messung der pHabhängigen Veränderungen des Maximums der Fluoreszenzemission simultan erfolgt. Dies erfolgt in an sich bekannter Weise dadurch, daß man innerhalb des in Frage kommenden Frequenzbereichs gleichzeitig die Intensität in zwei « Lichtfenstern » misst, also z. B. zwischen 420 und 440 sowie ferner zwischen 500 und 580 mm. Das Verhältnis beider Werte ist ein Maß für die Frequenz des Maximums der Fluoreszenzemission, die Frequenz wiederum ein Maß für den pH-Wert. Da die Substanzen, die diese Eigenschaften aufweisen, nämlich z. B. das 2,3-Dicyano-1,4-Hydrochinon (DH) jedoch nicht membrangängig sind, kam man seither nicht auf die Möglichkeit, sie in größerem Maßstab hierfür einzusetzen, weil, sofern man in einer Lösung des DH Zellen inkubierte, dieses nicht durch die Zellwände hindurch in das Innere der Zellen hineinwanderte und sich dort anreicherte. Um nun dennoch Stoffe mit der genannten Frequenzabhängigkeit des Emissions-peaks so zu verändern, daß sie membrangängig werden, sieht die Erfindung in Analogie zu der für die Anreicherung von Fluorescein in den Zellenverwendeten Methode eine Derivatisierung vor, d. h. eine Umwandlung in einen Stoff, der die Tendenz hat, aus einer Lösung durch die Zellwand hindurch in das Zellinnere überzugehen. Gleichzeitig muß dies eine solche Verbindung der Fluoreszenz-Substanz sein, die im Innern der Zelle durch die dort vorhandenen Enzyme wiederum so gespalten wird, daß die ursprüngliche fluoreszente Substanz entsteht.

Als eine solche fluoreszierende Substanz, die in der beschriebenen Weise pH-abhängig fluoresziert, wird gemäß einer vorteilhaften Weiterbildung der Erfindung das 2,3-Dicyano-1,4-Hydrochinon (auch : 2,3-Dicyanohydrochinon) verwandt. Diese Substanz ist an sich bekannt (siehe CRC Handbook of Chemistry and Physics, 54. Aufl., S. D-117 ; dort in anderer Nomenklatur bezeichnet als 3,6-Dioxyphtalic dinitrile (engl.)). Diese Substanz hat die gewünschten Eigenschaften der Fluoreszenz. Das Maximum der Emission ist pH-abhängig. Es ergibt sich zudem der Vorteil, daß die Fluoreszenz in einem Frequenzbereich auftritt, der wesentlich unterschiedlich ist von dem, bei dem andere für analytische Zwecke gewichtige Fluoreszenzen auftreten (z. B. solche, die für die DNA-Konzentrationsbestimmung wichtig sind). Man kann also auf diese Weise nicht nur die Fluoreszenz, die für die pH-Bestimmung wichtig ist, sondern auch noch andere Fluoreszenzen und damit mehr als nur einen Parameter gleichzeitig messen.

Eine Möglichkeit, die Membrangängigkeit zu erhalten, ist die Acylierung. Hierfür ein Beispiel ist die Veresterung. Dann sind es die in einer Zelle vorhandenen Esterasen, die nach dem Durchtritt des Esters durch die Zellwand durch Hydrolyse den Indikator wieder spalten und damit zur Wirksamkeit bringen.

Die Veresterung z. B. mit Essigsäure ergibt im Falle von 2,3-Dicyano-1,4-Hydrochinon das aus J. Chem. Soc. 1948, 303 bekannte 1,4-Diacetoxy-2,3-Dicyano-benzol (DDB). Werden Zellen in einer Lösung von DDB inkubiert, nehmen sie dies schnell und in hohem Maße auf. Es entsteht in der Zelle durch Hydrolyse wieder das 2,3-Dicyano-1,4-hydrochinon. Dabei stellt sich heraus, daß das in der Zelle und auch in der Lösung durch Hydrolyse aus DDB entstehende DH für pH-Messungen in etwa dem doppelten pH-Bereich brauchbar ist als man seither wusste, offenbar weil die für die Fluoreszenz Aktivität verantwortliche phenolische Struktur der Verbindung im Zellinnern und in Lösung durch Oxidation nicht entscheidend verändert werden kann. Während man seither davon ausging, daß eine pH-Abhängigkeit der Fluoreszenz bei 2,3-Dicyano-1,4-Hydrochinon nur zwischen $pH = 6$ und $pH = 8$ gegeben sei, so ergibt sie sich bei dem erfindungsgemäßen Verfahren zwischen $pH = 3$ und $pH = 9$ und somit für alle biochemisch wichtigen Werte.

Das neue 1,4-Diacetoxy-2,3-Dicyano-benzol ist, wie der Name besagt, so aufgebaut, daß sich an der 1. und 4. Stelle des Benzolsrings je eine Acetoxygruppe und an der 2. und 3. Stelle je eine Cyangruppe befindet. Die 5. und 6. Stellen sind mit H-Atomen besetzt. Die Erfindung schließt aber auch solche Substanzen mit ein, bei denen diese Stellen durch andere Gruppen oder Verbindungen (einschließlich der Anbindung weiterer Benzolringe) besetzt sind, durch die die genannten Aktivitäten (Fluoreszenz nach Hydrolyse in der Zelle ; gute Migration in die Zelle) nicht beseitigt oder sogar noch erhöht werden.

Anstelle der Essigsäure kann man zur Acylierung des Hydrochinons auch Buttersäure oder eine N-geschützte Aminosäure verwenden. Man erhält dann die ebenfalls neuen Substanzen 1,4-Dibutyryloxy-2,3-dicyanobenzol bzw. 1,4-Di-(tert-butyloxycarbonyl-1-alanyl-oxy)-2,3-dicyano-benzol als membrangängige Verbindungen des 2,3-Dicyanohydrochinons.

In seiner allgemeinen Form ermöglicht es dieses Verfahren, außerdem die Konzentration und die Kinetik von Enzymen in einzelnen Kompartimenten einer Zelle festzustellen. Dazu bewirkt man den Transport durch die Zellwand durch Ankupplung einer solchen Gruppe, die nur von einem Enzym in einem bestimmten Kompartiment der Zelle abgespalten wird. Die Fluoreszenz ergibt sich innerhalb der Zelle also nur dort, wo auch dieses Enzym vorhanden ist.

Ein Ausführungsbeispiel der Erfindung wird im folgenden unter Benzugnahme auf die beigefügten Zeichnungen näher beschrieben. Es stellen dar :

Figur 1 das Exzitationsspektrum des 2,3-Dicyano-1,4-Hydrochinons (DH) ;

Figur 2 das Emissionsspektrum des 2,3-Dicyano-1,4-Hydrochinons (DH) ;

Figur 3 die Frequenzabhängigkeit der Lage des Maximums der Fluoreszenz für DH, DDB und DH nach Hydrolyse in der Zelle ;

Figur 4 einige gemäß der Erfindung gemessene pH-Verteilungen.

Ausgangssubstanz, d. h. im Sinne des Patentanspruches 1 : Fluoreszierende Substanz ist das 2,3-Dicyano-1,4-Hydrochinon (DH). Es hat folgende Strukturformel :

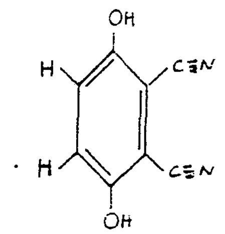

(1)

3,2 g des frisch umkristallisierten DH werden in 30 ml $H_2O$ + 2,4 g NaOH unter Argon-Atmosphäre bei 0 °C mit 4,7 ml Acetanhydrid versetzt. Der sich rasch abscheidende Niederschlag wird nach vier Minuten abfiltriert, mit Wasser gewaschen und getrocknet. Nach dem Trocknen im Vakuum wird der Niederschlag aus Äthanol umkristallisiert. Die Ausbeute beträgt bei den genannten Ausgangsmengen 3,09 g. Man erhält auf diese Weise 1,4-Diacetoxy-2,3-Dicyano-benzol $C_{12}H_8N_2O_4$ (DDB). Dieses hat folgende Strukturformel :

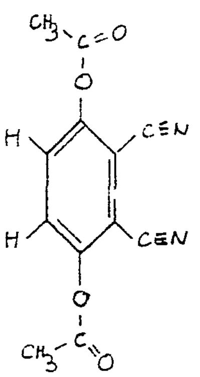

(2)

Die geschilderte Reaktion lief wie folgt ab :

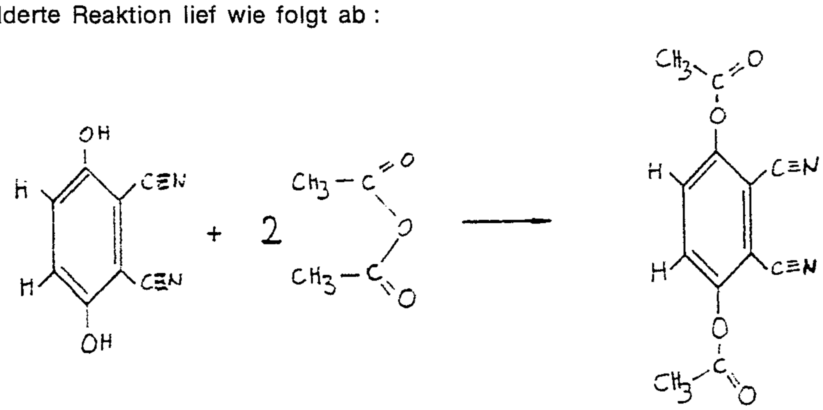

(3)

Die Reaktion ist eine Acetylierung.

Das durch diese Reaktion erhaltene DDB hat einen Fließpunkt von 165 bis 167 °C. Es ist chromatographisch rein in Dichloräthan/Heptan/Methanol 90 : 10 : 5. Als Analyse ergibt sich :

C 59,02 H 3,30 M 11,47 berechnet
C 58,95 H 3,33 M 11,48 gefunden.

Eine Lösung des DDB wird wie folgt erstellt :
1 mg DDB wird in 1 ml DMSO (Dimethylsulfoxid) gelöst.
Eine für die Messung geeignete Zellsuspension wird wie folgt erstellt :
250 µl einer Zellsuspension der Konzentration $1 \times 10^7$ Zellen/ml in einer 0,95 %-iger Kochsalzlösung, die mit 10 mMol Trishydroximethyaminomethan/HCl auf pH = 7,4 gebracht wurde, werden mit 5 µl der DDB-Lösung zusammengegeben. Das entspricht einer Konzentration von 20 µg DDB Substanz pro ml Zellsuspension.

Die DDB-haltige Zellsuspension wird 10 Minuten bei Zimmertemperatur inkubiert.

Dann wird die Zellsuspension in den Zell-Vorratsbehälter eines Durchflußcytometers gegeben und die Fluoreszenz der Einzelzellen in zwei Lichtfenstern gemessen.

Die Fluoreszenz der Einzelzellen kann entsprechend der Spektren in Fig. 1 im Bereich 300-410 nm angeregt werden. Dabei ergeben sich Emissionssprektren für die inzwischen im Zellinnern hydrolisierte und angereicherte Substanz, nämlich für das 2,3-Dicyanohydrochinon, die denen der Fig. 2 ähnlich sind, welche in freier Lösung gemessen wurden. Es ist ersichtlich, daß die Lage der Maxima eindeutig vom pH-Wert abhängig ist.

Infolge der guten Membrangängigkeit des DDB hängt die Menge DDB und die Durchtrittsgeschwindigkeit vom Unterschied der Konzentrationen dieser Substanz außerhalb und innerhalb der Zelle ab. Da aber das in die Zelle eingetretene DDB dort enzymatisch zu DH hydrolisiert (gespalten) wird, besteht so lange ein Konzentrationsgefälle für DDB, wie überhaupt die Lösung, in der die Zellen inkubiert sind, noch eine nennenswerte Konzentration an DDB aufweist. Die oben bei den Versuchsbedingungen angegebenen 10 Minuten reichen vollständig aus, um genügend DH für die Messung der Fluoreszenz in der Zelle anzureichern.

Die in Fig. 2 dargestellte Verschiebung der Maxima der Emissionsspektren bei verschiedenen $pH_I$-Werten wird meßtechnisch dadurch festgehalten, daß man in zwei « Lichtfenstern » die Fluoreszenz misst und den Quotient der Meßwerte bildet.

Diese beiden « Lichtfenster » sind inf Fig. 2 eingezeichnet und mit $F_1$ sowie mit $F_2$ bezeichnet. Der Quotient der beiden Meßwerte ist bei den gezeigten typischen Kurvenverläufen ein Maß für die Lage des Maximums und damit direkt mit dem $pH_I$-Wert in Beziehung zu setzen. Die Einzelheiten dieses Meßvorgangs bedürfen keiner weiteren Darstellung, da sie dem Fachmann geläufig sind.

Die Abhängigkeit der Frequenz, bei der das Maximum des Emissionssprektrums auftritt, vom pH-Wert ist in Fig. 3 dargestellt. Dort sind drei Kurven eingezeichnet. Die erste Kurve (dargestellt durch kleine Kreise) zeigt die Abhängigkeit der Wellenlänge des Emissions-peaks für die ursprüngliche Substanz DH vor der Veresterung und vor dem Eintritt in das Zellinnere. Zwischen pH 5,5 und pH 8 ist ein annähernd linearer Anstieg zu erkennen, der die Eignung der Substanz für eine solche Messung zeigt.

Kurve 2 (kleine Dreiecke) zeigt die Messungen für die veresterte Substanz DDB, die in das Zellinnere eindringt. Wie ersichtlich, bewirkt die Veresterung, daß das Emissionsspektrum und die Lage des peaks unabhängig vom pH-Wert ist.

Die Kurve 3 (Punkte) zeigt die Messung nach dem Eindringen von DDB in die Zelle und dem Wiederentstehen von DH durch Hydrolyse. Es zeigt sich erneut die Abhängigkeit der Frequenz, bei der das Emissionspeak auftritt, vom pH-Wert, und — was besonders überrascht — in einem wesentlich weiteren Arbeitsbereich, nämlich zwischen pH 3 und pH 9. Das ist, wie bereits erläutert, wahrscheinlich darauf zurückzuführen, daß beim Entstehen von DH in der Zelle durch Hydrolyse aus DDB nicht sofort dadurch wieder eine teilweise Unwirksamkeit eintritt, daß die für die Fluoreszenz verantwortlichen OH-Gruppen (an Stelle 1 und Stelle 4) des DH schon wieder teilweise oxidiert werden.

Die genannten alternativ verwendbaren membrangängigen Verbindungen des DH werden wie folgt hergestellt :

a) 1,4-Dibutyryloxy-2,3-dicyanobenzol :

Zu 1,6 g 2,3-Dicyanohydrochinon in 30 ml 1N NaOH und 10 ml Dioxan gibt man 4,1 ml Buttersäureanhydrid. Nach 15 Minuten Rühren verdünnt man mit Wasser und extrahiert die Reaktionsmischung mit Essigester. Die abgetrennte organische Phase wird nacheinander mit 0,1N NaOH und Wasser gewaschen und im Vakuum eingedampft. Der Rückstand wird aus Äthanol umkristallisiert. Ausbeute 2,3 g (77 % d. Th.). Schmelzpunkt 86-87 °C.

$C_{16}H_{16}N_2O_4$ (300.3)

Berechnet C 63,99 H 5,37 N 9,33
Gefunden C 64,00 H 5,38 N 9,34

Das 1,4-Dibutyryloxy-2,3-dicyanobenzol hat folgende Strukturformel :

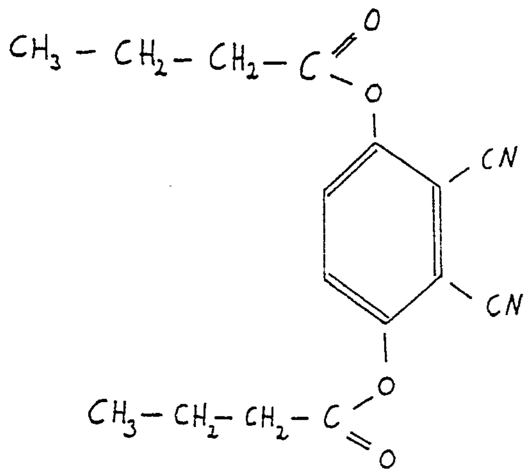

b) 1.4 Di-(tert-butyloxycarbonyl-alanyl-oxy)-2,3-dicyanobenzol :

4,7 g tert-butyloxycarbonyl-1-alanin und 1,6 g 2,3-Dicyanohydrochinon werden in 100 ml Essigester unter Eiswasserkühlung mit 5,2 g Dicyclohexylcarbodiimid versetzt. Nach 12 Stunden Rühren wird vom ausgefallenen Harnstoff abfiltriert, das Filtrat im Vakuum eingedampft und der Rückstand aus Äthanol umkristallisiert. Ausbeute 4,2 g (84 % d. Th.). Schmelzpunkt 164-167 °C (Zers.).

$C_{24}H_{30}N_4O_8 \cdot H_2O$ (520,52)

| | | | |
|---|---|---|---|
| Berechnet | C 55,38 | H 6,20 | N 10,76 |
| Gefunden | C 55,34 | H 6,12 | N 10,80 |

Das 1.4 Di-(tert-butyloxycarbonyl-alanyloxy)-2,3-dicyanobenzol hat folgende Strukturformel :

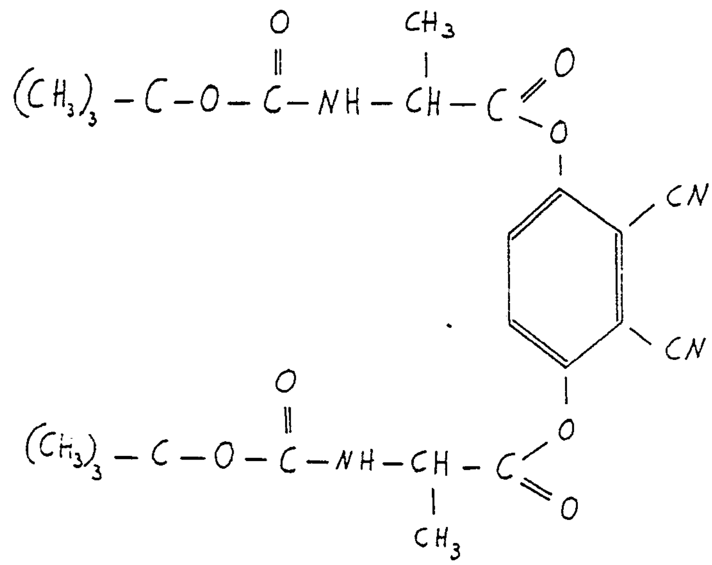

Fig. 4 erläutert nun den diagnostischen Wert der Messung des $pH_i$-Wertes für Zellpopulation. Fig. 4 zeigt drei Verteilungskurven, und zwar Kurve 1 (schwarze Punkte) für gesunde Knochenmarkzellen von Ratten, Fig. 2 (kleine Kreuze) für Ehrlich Ascites Tumorzellen und Fig. 3 (kleine Dreiecke) für menschliche Eierstock-Krebszellen.

Wie ersichtlich, ist für alle drei Arten die pH-Verteilungskurve der Zellpopulation eindeutig unterschiedlich. Die Messung solcher pH-Verteilungskurven von Einzelzellen wird erst durch das erfindungsgemäße Verfahren bzw. die gefundene Substanz DDB ermöglicht. Es eröffnen sich damit neue Wege der Diagnostik.

**Patentansprüche**

1. Verfahren zur Bestimmung des pH-Wertes im Inneren einer Zelle durch Messung der Emission einer fluoreszierenden Substanz, die von der Zelle durch Inkubation in einer Lösung aufgenommen wird,

die ein membrangängiges Derivat dieser Substanz enthält, und die in der Zelle durch enzymatische Spaltung aus diesem Derivat freigesetzt wird, dadurch gekennzeichnet, daß als fluoreszierende Substanz eine solche ausgewählt wird, bei der die Frequenz des Maximums der Emission pH-abhängig ist und daß die Frequenz gemessen wird, bei der das Emissions-Maximum auftritt.

2. Verfahren nach Anspruch 1, dadurch gekennzeichnet, daß das membrangängige Derivat der fluoreszierenden Substanz ein Ester derselben ist.

3. Verfahren nach Anspruch 1, dadurch gekennzeichnet, daß die fluoreszierende Substanz 2,3-Dicyano-1,4-Hydrochinon oder ein in 5 und/oder 6 Stellung substituiertes Derivat dieser Substanz ist.

4. Verfahren nach Anspruch 2 oder 3, dadurch gekennzeichnet, daß die eingesetzte Verbindung der fluoreszierenden Substanz 1,4-Diacetoxy-2,3-dicyano-benzol oder 1,4-Dibutyryloxy-2,3-dicyanobenzol oder 1,4-Di(-tert-butyloxycarbonyl-1-alanyloxy)-2,3-dicyano-benzol ist.

5. 1,4-Dibutyryloxy-2,3-dicyano-benzol.

6. 1,4-Di(-tert-butyloxycarbonyl-1-alanyloxy)-2,3-dicyano-benzol.

## Claims

1. Process for determining the pH-value in the interior of a cell by measuring the emission of a fluorescent substance, which is introduced into the cell by incubation in a solution, said solution containing a membrane-permanent derivative of said substance, and said substance being recovered in the cell from this derivative by enzymatic cracking, characterized in that as a fluorescent substance such a substance is selected, with which the frequency of the maximum of emission is pH-dependent, and that the frequency is measured, which occurs at this maximum of emission.

2. Process in accordance to claim 1, characterized in that the membrane-penetrating derivative of the fluorescent substance is an ester of the same.

3. The process as claimed in claim 1 wherein said fluorescent substance is 2,3-dicyano-1,4-hydroquinone or a derivative of the same having substituents in the fifth and/or sixth position.

4. The process as claimed in claim 2 or 3 wherein said introduced substance is 1,4-diacetoxy-2,3-dicyanobenzene or 1,4-dibutyryloxy-2,3-dicyanobenzene or 1,4-di(-tert-butyloxycarbonyl-1-alanyloxy)-2,3-dicyanobenzene.

5. 1,4-dibutyryloxy-2,3-dicyanobenzene.

6. 1,4-di(-tert-butyloxycarbonyl-1-alanyloxy)-2,3-dicyanobenzene.

## Revendications

1. Procédé de détermination du pH à l'intérieur d'une cellule par mesure de l'émission d'une substance fluorescente qui est absorbée par la cellule par incubation dans une solution contenant un dérivé de cette substance capable de franchir une membrane de la cellule et qui est libérée de ce dérivé dans la cellule par dissociation enzymatique, caractérisé en ce qu'on choisit comme substance fluorescente une substance pour laquelle la fréquence du maximum de l'émission est dépendante du pH, et que la fréquence mesurée est celle pour laquelle se produit le maximum de l'émission.

2. Procédé selon la revendication 1, caractérisé en ce que le dérivé de la substance fluorescente ayant la capacité de franchir la membrane est un ester de cette substance.

3. Procédé selon la revendication 1, caractérisé en ce que la substance fluorescente est la 2,3-dicyano-1,4-hydroquinone ou un dérivé substitué en position 5 et/ou en position 6 de cette substance.

4. Procédé selon la revendication 2 ou 3, caractérisé en ce que le composé utilisé de la substance fluorescente est le 1,4-diacétoxy-2,3-dicyanobenzène ou le 1,4-dibutyryloxy-2,3-dicyanobenzène ou le 1,4-dibutyryloxy-2,3-dicyanobenzène ou le 1,4-di(-tert-butyloxy-carbonyl-1-alanyloxy)-2,3-dicyano benzène.

5. 1,4-dibutyryloxy-2,3-dicyanobenzène.

6. 1,4-di(-tert-butyloxycarbonyl-1-alanyloxy)-2,3-dicyano-benzène.

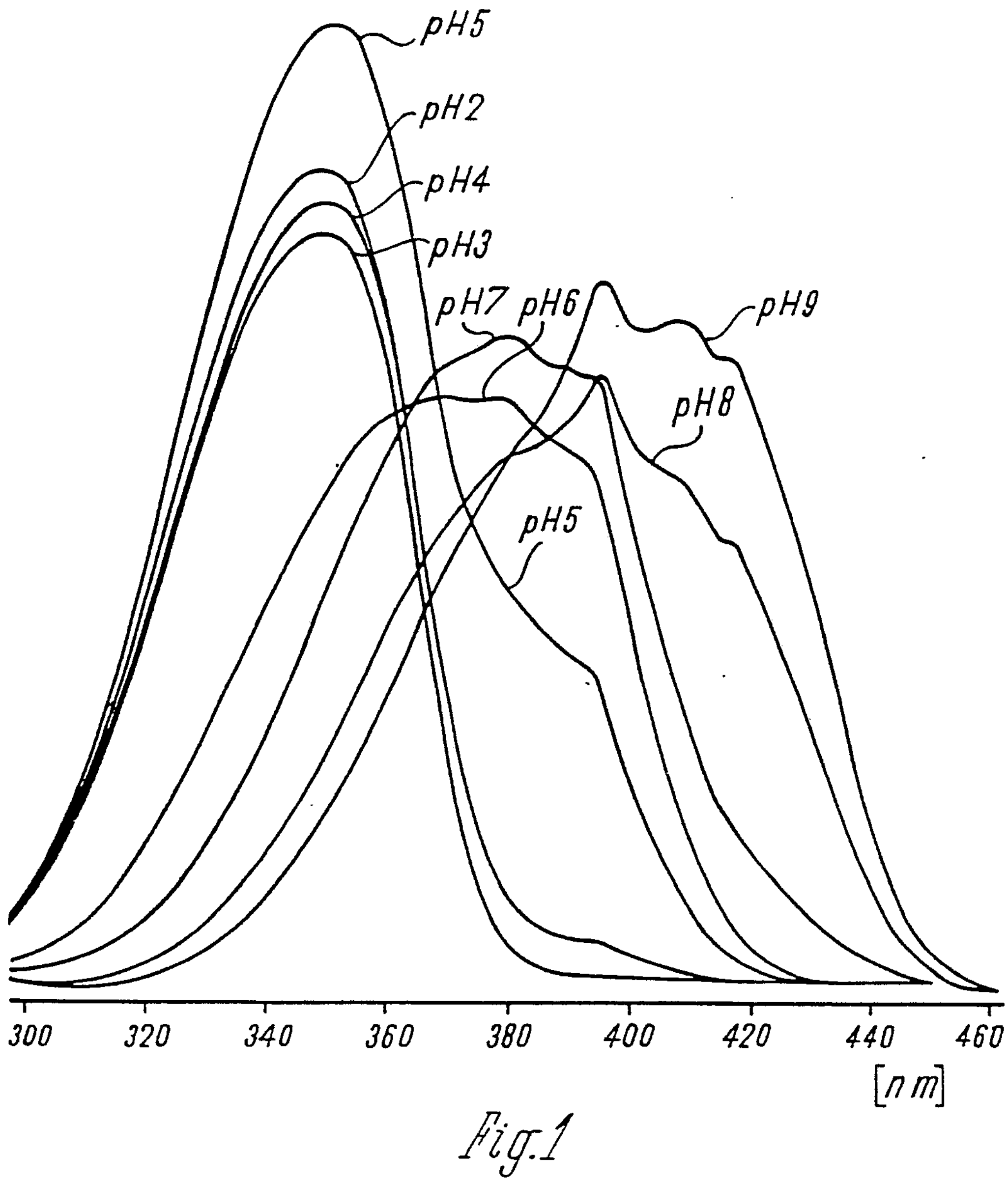
pH5
pH2
pH4
pH3
pH7
pH6
pH9
pH8
pH5
300
320
340
360
380
400
420
440
460
[nm]

Fig.1

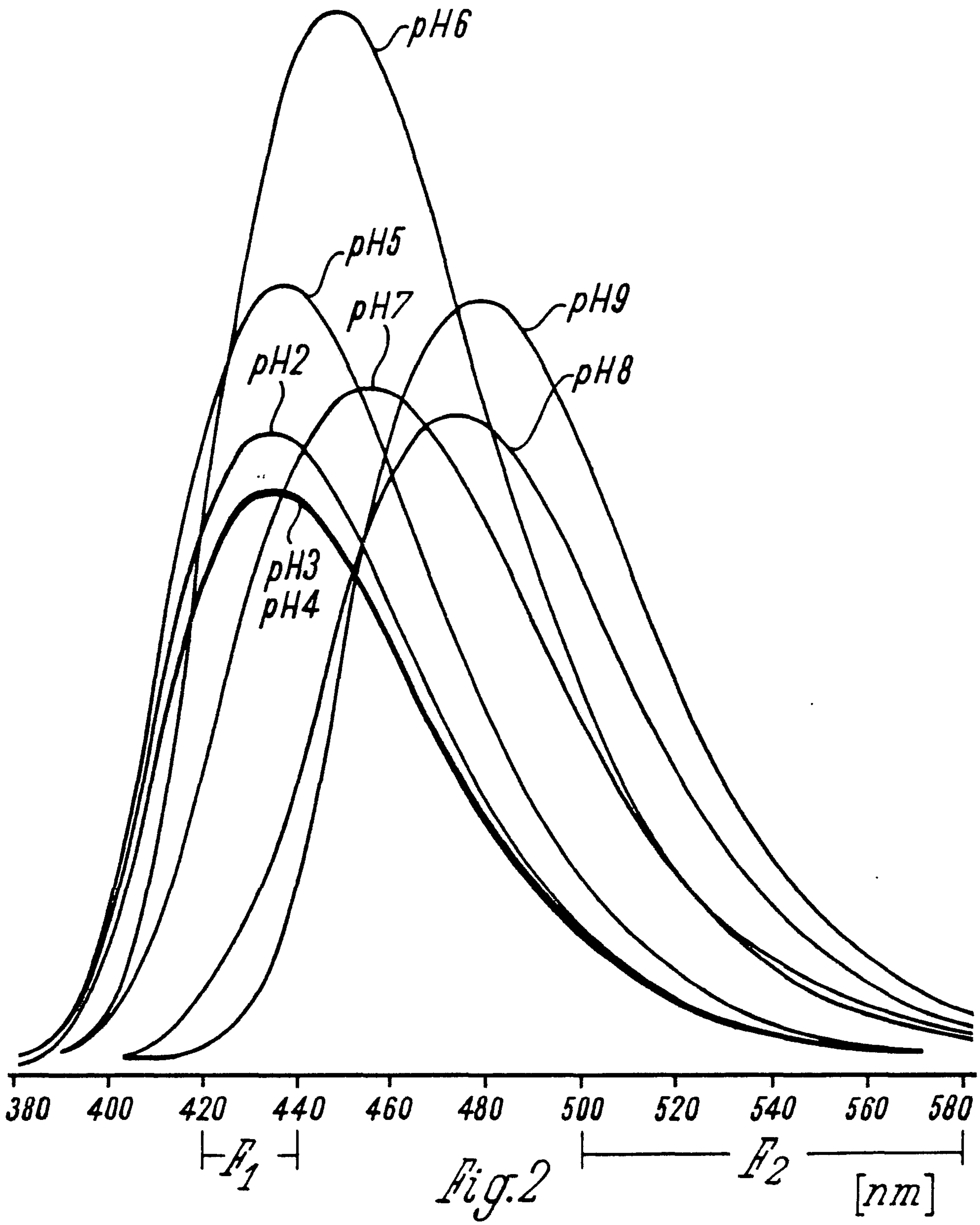
pH6
pH5
pH7
pH9
pH2
pH8
pH3
pH4
380
400
420
440
460
480
500
520
540
560
580
F1
F2
[nm]

Fig.2

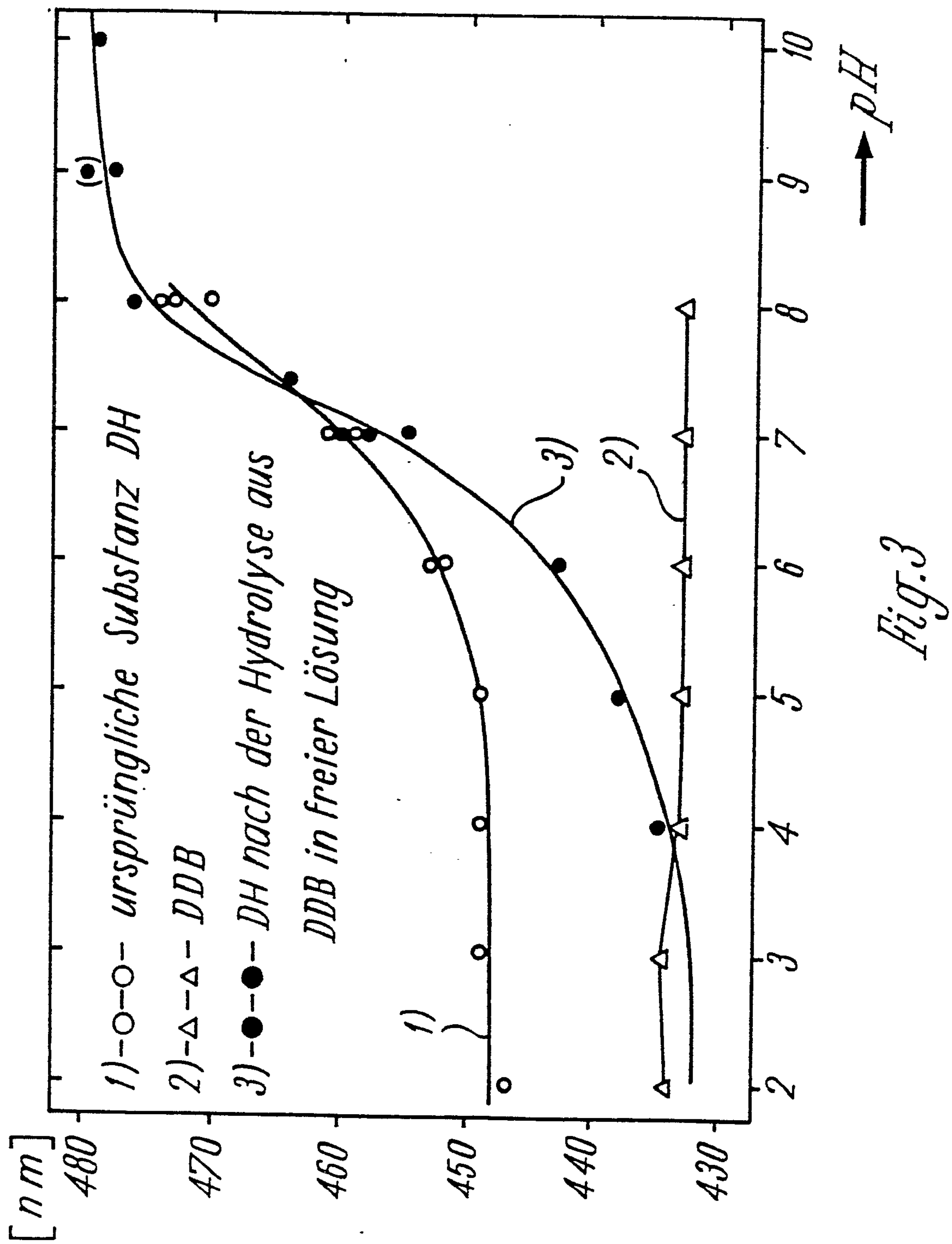
1)–o–o– ursprüngliche Substanz DH
2)–△–△– DDB
3)–●–●– DH nach der Hydrolyse aus DDB in freier Lösung
[nm]
480
470
460
450
440
430
2
3
4
5
6
7
8
9
10
pH
1)
2)
3)

Fig.3

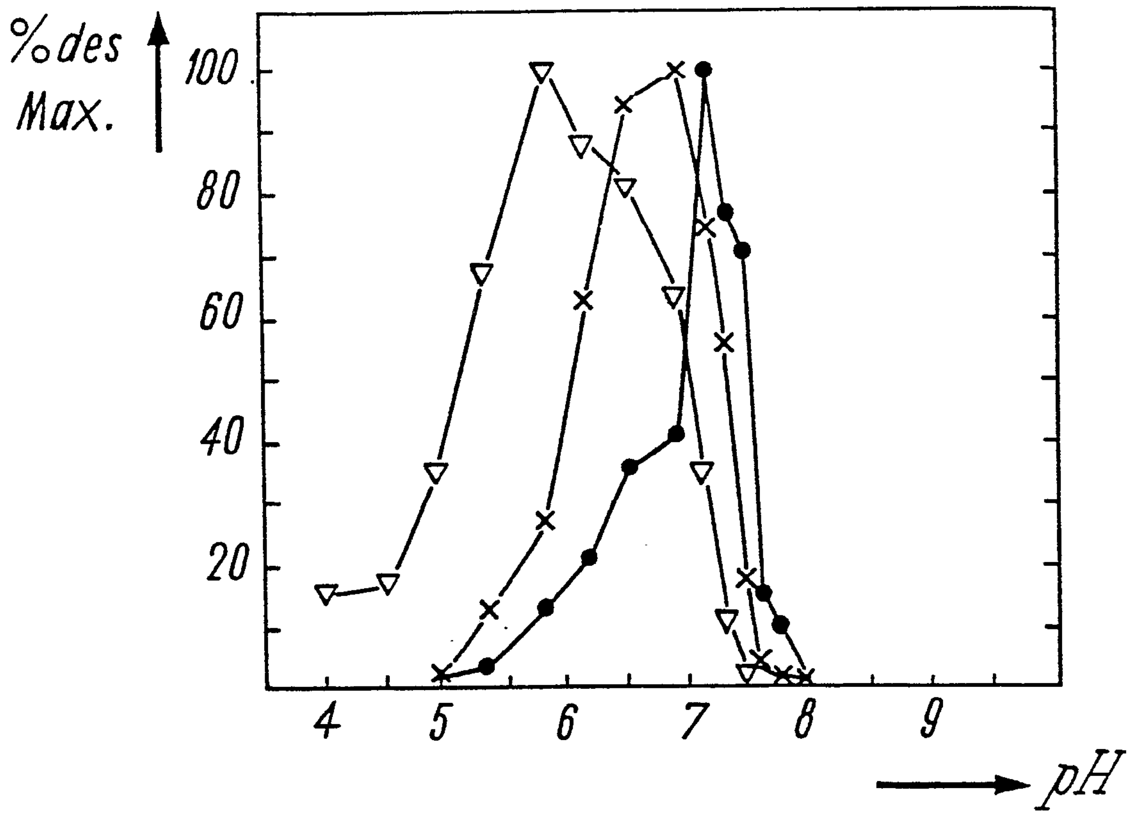


1) —•—•— Knochenmark von Ratten

2) —×—×— Ehrlich Ascites Tumor der Maus

3) —▽—▽— Hum. Eierstock-CA

Fig.4